# DEMANDE DE BREVET EUROPEEN

(11) **EP 4 371 583 A1**
(43) Date de publication de la demande: **22.05.2024**
(21) Numéro de dépôt: 23206089.7
(22) Date de dépôt: 26.10.2023
(51) Int. Cl.: A61L 27/06, A61L 27/36, A61L 27/50, B33Y 80/00, A61L 31/02, A61L 31/14

(54) **NOUVEAU DISPOSITIF DE RÉGÉNERATION OSSEUSE**

(30) Priorité: 21.11.2022 FR 2212116
(71) Demandeur: BIOTECH DENTAL, 13300 Salon-de-Provence (FR)
(72) Inventeur: KELLER, Pierre, 67000 Strasbourg (FR)
(74) Mandataire: Barbot, Willy

(57) **Abrégé**

La présente invention concerne un dispositif (1) destiné à la régénération osseuse qui comprend une plaque de recouvrement en titane de grade 1 ou de grade 2 réalisée sur mesure qui recouvre un défaut osseux dont la forme permet de recréer la surface de l'os préalablement à l'apparition du défaut osseux et, éventuellement, au moins un moyen de fixation.

## Description

La présente demande de brevet revendique la priorité de la demande de brevet Français FR2212116 déposée en date du 21 novembre 2022.

### DOMAINE DE L'INVENTION

La présente invention est relative au domaine des dispositifs destinés à la régénération osseuse.

### ART ANTERIEUR

Une destruction du tissu osseux peut parfois s'opérer avec une origine qui peut être infectieuse, tumorale ou traumatique. Cette destruction est normalement suivie d'une cicatrisation osseuse. Maintenant, et dans certains cas, cette cicatrisation osseuse peut s'avérer insuffisante pour régénérer l'intégrité du tissu détruit avec l'apparition de défauts osseux. De tels défauts osseux correspondent à des cavités en lieu et place de l'os détruit.

Face à de tels défauts osseux, il est possible d'utiliser des biomatériaux permettant de combler un défaut osseux et de favoriser la cicatrisation osseuse en son sein.

En règle générale, le biomatériau utilisé est constitué soit de matériau de substitution osseuse synthétique (par exemple des granules d'hydroxyapatite), soit de particules d'os. Une fois l'évidement correspondant au défaut osseux rempli par ce biomatériau, cet évidement est fermé par un dispositif de recouvrement qui permet de diriger la croissance de ce biomatériau.

Deux types de dispositifs de recouvrement ont pu être développés dans l'art antérieur.

Un premier type de dispositif correspond aux membranes de recouvrement utilisées depuis le début des années 60 en chirurgie osseuse orthopédique. Ces membranes tissées ou non tissées peuvent présenter de multiples structures, que ce soit sous la forme de membranes simples (ex : brevet US 4 961 707), de membranes à base de multicouches synthétiques (ex : brevet US 4,816,339), de treillis en titane (ex : modèle d'utilité RU 152119 U1), voire avec des compositions hybrides (synthétique et titane).

Ces membranes de recouvrement présentent des caractéristiques intéressantes, puisque :
1- Elles sont faciles à poser par le praticien et stabilisent la greffe osseuse et le caillot.
2- Elles s'opposent à la résorption de la greffe qui, sans membrane, peut perdre jusqu'à 25 % de son volume après 4 mois.
3- Elles préviennent la prolifération des cellules à partir de la muqueuse de recouvrement et favorisent donc la migration des cellules issues des espaces médullaires dans le caillot habitant le site à régénérer.
4- Elles peuvent enfin être retirées sans difficulté (maintenant certaines sont résorbables).

Maintenant, ces membranes de recouvrement présentent comme désavantage de présenter une très grande souplesse qui, si elle facilite leur pose, ne leur permet pas de maintenir le volume de régénération tel que délimité par le praticien au moment de leur pose dès lors qu'elles sont soumises à des contraintes telles que celles résultant d'une mastication du patient sur cette zone.

Un second type de dispositifs de recouvrement développé à partir des années 90 a consisté lui en des plaques de recouvrement en titane (rigides et perforées) telles que décrites dans le brevet EP 3 294 222 B1 ou le brevet RU 2 748 200 C1.

Ces plaques de recouvrement présentent également des caractéristiques intéressantes, puisque :
1- Elles sont d'une pose accessible, même si nécessitant un savoir-faire certain, du fait de leurs larges mailles qui permettent leur mise en forme par le praticien.
2- Elles s'opposent partiellement à la résorption de la greffe
3- Leur rigidité permet de délimiter stablement dans le temps le volume de régénération osseuse, même lors d'épisodes de mastication du patient.

Maintenant, ces plaques de recouvrement présentent comme désavantage, outre leur pose plus délicate que les membranes et la résorption de greffe qui peut lui être associée, de ne pas bloquer la prolifération des cellules à partir de la muqueuse de recouvrement et surtout de présenter une structure très ouverte (maillée) qui peut rendre difficile leur retrait après ostéo-régénération du fait du risque d'arrachement des tissus qui auraient pu se développer et/ou s'ancrer au sein de leurs mailles.

Ces deux dispositifs présentent donc des avantages et inconvénients mutuels et, à priori, opposés.

Par le passé, certains ont bien essayé de coupler ces deux dispositifs pour bénéficier de leurs avantages respectifs (ex : brevet US 4 961 707), mais c'est au prix d'une bien plus grande complexité.

### SOMMAIRE DE L'INVENTION

Face à cette problématique, les inventeurs ont maintenant développé une plaque de recouvrement qui présente une rigidité suffisante de sorte à lui conférer une forme corrigeant le défaut osseux et à résister aux épisodes de mastication du patient, tout en présentant simultanément une élasticité lui permettant d'être ajustée simplement au défaut osseux lors de sa pose part le praticien.

Pour se faire, les inventeurs ont développé une membrane de recouvrement fabriquée spécifiquement en titane de grade 1 ou 2.

Le titane est un métal de transition brillant avec une faible densité et une résistance élevée. Le titane « pur » est ainsi plus résistant que les aciers ordinaires à faible teneur en carbone, tout en étant 45 % plus léger. L'intégration du titane au sein de différents alliages permet de leur associer des propriétés particulièrement intéressantes. Ainsi, des alliages de titane sont généralement utilisé pour la plupart des applications avec de petites quantités d'aluminium et de vanadium, généralement 6% et 4% respectivement, en poids.

Le titane solide et ses alliages peuvent se trouver sous la forme de deux structures cristallines, à savoir hexagonale compacte (phase α et au-dessous de 880 ⁰C pour le titane « pur ») et/ou cubique centrée (phase β ; au-delà de 880 ⁰C pour le titane « pur »). Ce dimorphisme est à la base d'une classification correspondant aux familles « *alpha* », « *alpha-bêta* » et « *bêta* » avec chacune des propriétés d'usage particulières. Ainsi, les plus hautes résistances mécaniques sont obtenues avec les alliages β alors que le titane « pur » appartient lui à la famille alpha. Maintenant, il est possible d'effectuer un traitement thermique sur le titane pur ou de l'un de ses alliages, on parle de « recuit ». Ce traitement correspond à une montée en température contrôlée du titane ou de l'un de ses alliages de sorte à entraîner une transition d'une structure cristalline en phase α vers une structure cristalline en phase β, la stabilité de cette transition pouvant varier.

Maintenant, une autre classification existe pour le titane, à savoir les « grades », laquelle est relative à la pureté du titane. Dans le détail, on parlera de titane « pur » pour les titanes de grade 1 à 4 et d'alliages de titane pour les titanes de grade 5 et au-delà. En fait, le titane n'est jamais strictement pur et on trouve toujours en son sein des traces d'autres éléments (oxygène et d'autres composants). Ainsi, le titane le plus pur est le titane de grade 1, mais il contient tout de même 0,18% d'oxygène. Le titane de grade 4 est le titane le moins pur (des 4 grades) et contient lui 0,4% d'oxygène.

Maintenant, au-delà d'une variation dans leur composition, les propriétés de ces différents grades de titane différent également, notamment en ce qui concerne la ductilité et la résistance mécanique.

Au final, le titane de grade 1 n'est tout simplement pas utilisé en implantologie car il est considéré comme trop ductile. Le titane de grade 2 a lui certes été utilisé par le passé, mais on lui préfère aujourd'hui le titane de grade 4 qui présente de meilleures propriétés mécaniques et qui sert de base aux plaques de recouvrement actuelles. A noter que le titane de grade 5 (alliage avec du niobium), dont la dureté est supérieure au titane de grade 4, est aujourd'hui utilisé pour certains composants prothétiques. Pour des revues sur l'utilisation actuelle du titane et de ses alliages en implantologie, on peut consulter LIU et al. (Regenerative biomaterials, vol.4, page 315 à 323, 2017) ou NICHOLSON (Prosthesis, vol.2, page 100 à 116, 2020).

Partant des deux types de dispositifs connus, l'inventeur n'a pas fait le choix d'une simple combinaison. A l'inverse, il a changé la structure de la plaque de recouvrement qu'il a choisi microperforée, et pas perforée, et en titane de grade 1 ou 2, laquelle plaque de recouvrement présente alors les avantages des deux types de dispositifs tout en se révélant simple de conception.

Aussi, un premier objet de l'invention se rapporte à un dispositif (1) destiné à la régénération osseuse qui comprend au moins une plaque de recouvrement dont la forme permet de recréer la surface d'un os, préalablement à l'apparition d'un défaut osseux, en recouvrant ledit défaut osseux tout en s'appuyant, au moins partiellement, sur l'os sain encadrant ledit défaut osseux, caractérisée en ce que ladite plaque de recouvrement est réalisée en titane de grade 1 ou de grade 2.

Pour faciliter la pose par le praticien et donner au patient le maximum de confort, la plaque de recouvrement est adaptée à l'anatomie du patient. Typiquement, cette plaque de recouvrement sera réalisé sur mesure, par exemple sur la base d'un moulage du défaut osseux à corriger.

Un deuxième objet de l'invention porte sur une plaque de recouvrement telle que définie précédemment.

Avantageusement, la plaque de recouvrement selon l'invention présente une épaisseur comprise entre 0,1 et 1,5mm, de préférence entre 0,3 et 1 mm
Avantageusement encore, la surface de la plaque de recouvrement présente des micro-perforation, lesquelles présentent un diamètre compris entre 10 et 500 µm, de préférence entre compris entre 50 et 100µm.

### DESCRIPTION DES FIGURES

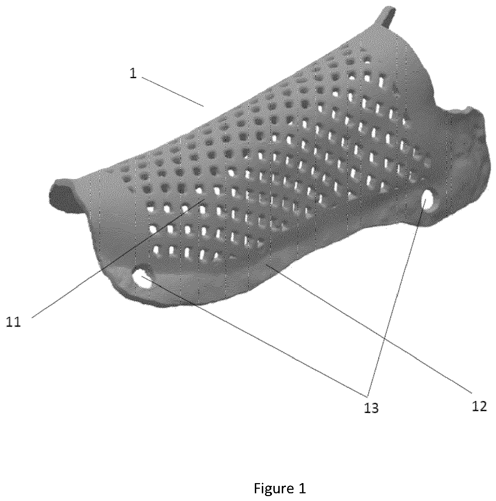

La figure 1 est une représentation d'un mode de réalisation d'une plaque de recouvrement d'un dispositif selon l'invention.

### DESCRIPTION DETAILLEE DE L'INVENTION

La plaque de recouvrement peut présenter une forme arrondie, rectangulaire ou, plus généralement, toute forme adaptée à la surface osseuse de destination. Maintenant, et dans le cas d'une plaque de recouvrement de forme rectangulaire, on prendra soin d'en arrondir les angles en vue de ne pas risquer de blesser le périoste ou les tissus mous.

Les dimensions de cette plaque de recouvrement sont telles qu'elle recouvre intégralement le défaut osseux avec, en outre, au moins 50 % de sa surface s'appuyant sur l'os sain encadrant ledit défaut osseux, de préférence au moins 10 % de sa surface.

Typiquement, le défaut osseux présente :
- une longueur comprise entre 5 et 200 mm, de préférence entre 10 et 100 mm ;
- une largeur comprise entre 3 et 20 mm, de préférence entre 5 et 10 mm ; et
- une profondeur comprise entre 5 et 20 mm, de préférence entre 8 et 15 mm.

Selon un mode de réalisation préféré, le défaut osseux est un défaut osseux de la mâchoire.

Pour présenter ses propriétés particulièrement avantageuses, la plaque de recouvrement du dispositif selon l'invention présente une épaisseur comprise entre 0,1 et 1,5mm, de préférence entre 0,3 et 1 mm.

Si la plaque de recouvrement peut être obtenu par toute méthode connue de l'homme du métier, que ce soit le moulage ou le fraisage, elle est préférentiellement fabriquée par impression 3D. Une telle impression 3D utilise typiquement une poudre métallique dont la mise en forme en plaque de recouvrement est assurée par un laser de puissance adaptée.

Selon un mode de réalisation préféré, la surface de la plaque de recouvrement présente des micro-perforations. Ces micro-perforations permettent l'apport d'éléments nutritifs à l'os en régénération sur le défaut osseux, en permettant à ces éléments nutritifs de traverser l'épaisseur de la plaque. Maintenant, leur taille empêche simultanément la colonisation de la plaque de recouvrement par des tissus (mous et/ou durs) et facilite donc la dépose de la plaque de recouvrement si cela était nécessaire.

Par micro-perforations, on entend des perforations qui présentent un diamètre compris entre 10 et 500 µm, de préférence entre compris entre 50 et 100µm.

Avantageusement, la plaque de recouvrement est préférentiellement réalisée en matériau plein (et donc sans micro-perforations) sur sa surface qui s'appuie sur l'os sain encadrant le défaut osseux.

Typiquement, cette surface réalisée en matériau plein constitue une bande qui forme tout ou partie du bord de la plaque de recouvrement. De préférence, la largeur de ce bord est comprise entre 0.5 et 5 mm, de préférence entre 1 et 2 mm.

Selon un autre mode de réalisation préféré, le dispositif selon l'invention comprend en outre au moins un moyen de fixation.

Typiquement, ledit moyen de fixation peut prendre la forme d'une broche, d'une vis, d'un clou ou d'une colle adaptée à l'os.

Avantageusement, ledit moyen de fixation prend la forme d'une vis et la plaque de recouvrement comprend alors de préférence un fraisage adapté à la tête de ladite vis. De préférence, le fraisage est positionné sur la plaque de recouvrement de sorte à coïncider avec une zone d'os sain, entourant le défaut osseux.

Avantageusement encore et dans le cas où la plaque de remplacement est destinée à la régénération osseuse d'un défaut osseux de la mâchoire, ladite plaque de recouvrement ne comprend aucun fraisage positionné du côté de la crête palatine ou de la cavité linguale.

En effet, les caractéristiques de la plaque de recouvrement en titane de grade 1 ou de grade 2 permettent un bon positionnement de celle-ci sans nécessiter de multiples fixations. Il est ainsi possible de se passer de fixation sur l'os de la mâchoire du côté de la crête palatine ou de la cavité linguale qui est difficile d'accès pour le praticien.

Les exemples qui suivent sont fournis à titre d'illustration et ne sauraient limiter la portée de la présente invention.

### Régénération osseuse d'un défaut osseux de la mâchoire en vue de la pose d'implants dentaires

Dans le cadre d'un défaut osseux au sein de la mâchoire, il est nécessaire de permettre une régénération de ce défaut osseux afin de pouvoir envisager la pose d'implants dentaires.

Pour se faire, une incision de la gencive est effectué de sorte à accéder à ce défaut osseux. Cette incision est opérée en opérant une élévation du lambeau de gencive consécutif de l'incision. Le praticien peut alors mettre en oeuvre, au niveau du défaut osseux, le matériau destiné à la régénération osseuse, notamment en utilisant la *Sausage Technique^{™}* du Pr ISTVAN URBAN.

La figure 1 montre une plaque de recouvrement (1) en titane de Grade 2 qui a été imprimée en 3D. Cette plaque de recouvrement (1) présente une surface centrale positionnée au-dessus du défaut osseux qui présente des micro-perforations (11). Cette plaque de recouvrement (1) présente encore une surfa ce périphérique qui prend la forme d'une bande en matériau plein (12) qui va s'appuyer sur l'os sain encadrant le défaut osseux.

Cette plaque de recouvrement (1) présente encore deux fraisages (13), situés dans la surface périphérique en matériau plein (12) de la plaque de recouvrement. Aucun de ces deux fraisages (13) n'est positionné du côté de la crête palatine ou de la cavité linguale par rapport au défaut osseux qu'il convient de régénérer.

Cette plaque de recouvrement (1) peut ainsi être posée et fixée par le praticien au moyen de vis se positionnant dans ces deux fraisages (13) et se fixant dans l'os sain de la mâchoire entourant le défaut osseux.

La fermeture du lambeau, sur la plaque de recouvrement, peut ensuite être réalisée par le praticien avec une suture (de préférence résorbable)
Après cicatrisation, la régénération osseuse peut s'opérer sous la plaque de recouvrement pendant une période qui peut aller jusqu'à plusieurs mois voire une année.

Après cette période de régénération, la plaque de recouvrement (1) peut être retirée sans compromettre la structure et donc la solidité de l'os régénéré. En effet, les microperforations de celle-ci empêche l'adhésion à celle-ci des tissus mous et durs.

De la sorte, le retrait de la plaque de recouvrement (1) selon l'invention ne compromet pas la structure et donc la solidifié de l'os régénéré. Il est alors possible de poser, dans cet os régénéré, des implants dentaires.

## Revendications

1. Un dispositif (1) destiné à la régénération osseuse qui comprend i) au moins une plaque de recouvrement dont la forme permet de recréer la surface d'un os, préalablement à l'apparition d'un défaut osseux, en recouvrant ledit défaut osseux tout en s'appuyant, au moins partiellement, sur l'os sain encadrant ledit défaut osseux**, caractérisée en ce que** ladite i) au moins une plaque de recouvrement :
- est réalisée en titane de grade 1 ou de grade 2.

2. Le dispositif selon la revendication 1, **caractérisé en ce que** le défaut osseux est un défaut osseux de la mâchoire.

3. Le dispositif selon l'une quelconque des revendications précédentes, **caractérisé en ce que** ladite i) au moins une plaque de recouvrement du dispositif selon l'invention présente une épaisseur comprise entre 0,3 et 1 mm.

4. Le dispositif selon l'une quelconque des revendications précédentes, **caractérisé en ce que** ladite i) au moins une plaque de recouvrement est fabriquée par impression 3D.

5. Le dispositif selon l'une quelconque des revendications précédentes, **caractérisé en ce que** la surface de ladite i) au moins une plaque de recouvrement présente des micro-perforations, lesquelles correspondent à des perforations qui présentent un diamètre compris entre 10 et 500 µm, de préférence entre compris entre 50 et 100µm.

6. Le dispositif selon l'une quelconque des revendications précédentes, **caractérisé en ce que** ladite i) au moins une plaque de recouvrement est réalisée en matériau plein sur sa surface qui s'appuie sur l'os sain encadrant le défaut osseux.

7. Le dispositif selon la revendication précédente, **caractérisé en ce que** la surface réalisée en matériau plein constitue une bande qui forme tout ou partie du bord de ladite i) au moins une plaque de recouvrement avec la largeur de ce bord étant comprise entre 0,5 et 5 mm, de préférence entre 1 et 2 mm.

8. Le dispositif selon l'une quelconque des revendications précédentes, **caractérisé en ce qu'**il comprend en outre ii) au moins un moyen de fixation.

9. Le dispositif selon la revendication précédente, **caractérisé en ce que ledit** ii) au moins un moyen de fixation prend la forme d'une vis et la plaque de recouvrement comprend alors un fraisage adapté à la tête de ladite vis.

10. Une plaque de recouvrement dont la forme permet de recréer la surface d'un os, préalablement à l'apparition d'un défaut osseux, en recouvrant ledit défaut osseux tout en s'appuyant, au moins partiellement, sur l'os sain encadrant ledit défaut osseux, **caractérisé en ce qu'**elle est réalisée en titane de grade 1 ou de grade 2.
